# EUROPEAN PATENT APPLICATION

(11) **EP 4 321 173 A1**
(43) Date of publication of application: **14.02.2024**
(21) Application number: 22784719.1
(22) Date of filing: 07.04.2022
(51) Int. Cl.: A61K 39/39, C07K 7/00

(54) **ADJUVANT ACTIVITY ENHANCER AND ADJUVANT COMPOSITION**

(30) Priority: 07.04.2021 JP 2021065379
(71) Applicant: Denka Company Limited, Tokyo 103-8338 (JP)
(72) Inventor: MITSUMATA, Ryotaro, Gosen-shi, Niigata 959-1695 (JP)
(74) Representative: Wächtershäuser & Hartz Patentanwaltspartnerschaft mbB
(86) International application number: PCT/JP2022/017302
(87) International publication number: WO 2022/215737

(57) **Abstract**

Provided are an adjuvant activity enhancer for enhancing immune strength of existing adjuvants, and an adjuvant composition containing the same. An adjuvant activity enhancer for CpG-ODN or squalene-containing emulsion, having an active ingredient of a peptide nucleic acid to which a cell-penetrating peptide is bound.

## Description

### Technical Field

The present invention relates to an adjuvant activity enhancer and an adjuvant composition containing the same.

### Background Art

Adjuvants have been used to enhance vaccine immunogenicity; however, aluminum salts have been the only adjuvants approved for use in humans for a long time, and the mechanism thereof had not been revealed until recently. However, nowadays various receptors in innate immunity including a Toll-like receptor (TLR) are discovered, and research and development on adjuvants have progressed rapidly.

Accordingly, in recent years, ligand molecules for a Toll-like receptor, such as monophosphoryl lipid A (MPL), CpG oligonucleotide (CpG-ODN), Flagellin, and PolyI:C, have been developed or put on the market as adjuvants in addition to emulsion adjuvants, which have been known for a long time. That is, as the mechanism of the innate immunity has been revealed, drug discovery of adjuvants based on the molecules and mechanism has become possible, and thereby many new adjuvants have been put into practical use. Mixed adjuvants obtained by mixing a plurality of adjuvants with different mechanisms of action have also been developed, and the development of these new adjuvants has made it possible to induce Th1 immunity, which could not be induced by conventional aluminum salts, resulting in further enhancement of immune induction.

Adjuvants are roughly categorized into those inducing Th1, Th2, and both, based on the types of immune induction, and aluminum salts are categorized as Th2 type, MPL, CpG-ODN, and PolyI:C as Th1 type, and squalene-containing emulsion and Flagellin as those inducing both Th1 and Th2. The Th1 and Th2 immune responses play a different role on host defense; Th1 immunity shows resistance to viruses and intracellular parasitic bacteria, and Th2 immunity to parasites and bacteria. That is, the quality of immunity to be induced by a vaccine is different for each infection to be targeted, and hence, there is a need to select an appropriate adjuvant.

There are two points to select an adjuvant: one of them is the quality of immunity to be induced as described above; and the other is immune strength, that is, whether or not it is possible to enhance immune induction up to the defense level. Therefore, there is a need to select an adjuvant so that appropriate quality and strength of immunity can be obtained for each infection to be targeted or vaccine antigen.

The inventors of the present application have reported on a novel adjuvant having a peptide nucleic acid to which a cell-penetrating peptide (CPP) is bound in Patent Literature 1; however, it has not been reported on how this adjuvant effects any qualitative and action changes on the peptide nucleic acids to which a cell-penetrating peptide is bound when combined with another adjuvant.

### Citation List

### Non Patent Literature

Patent Literature 1: WO 2020/246584

### Summary of Invention

### Technical Problem

The present invention relates to providing an adjuvant activity enhancer for enhancing immune strength of existing adjuvants, and an adjuvant composition containing the same.

### Solution to Problem

As a result of intensive studies, the present inventors have found that, when a peptide nucleic acid to which a cell-penetrating peptide is bound is combined with CpG-ODN or squalene-containing emulsion, immune strength can be enhanced without changing the quality of immunity of the CpG-ODN or squalene-containing emulsion.

That is, the present invention is according to the following 1) to 7).
1) An adjuvant activity enhancer for CpG-ODN or squalene-comprising emulsion, having an active ingredient of a peptide nucleic acid to which a cell-penetrating peptide is bound.
2) A method for enhancing an adjuvant activity of CpG-ODN or squalene-comprising emulsion, in which a peptide nucleic acid to which a cell-penetrating peptide is bound is used in combination with the CpG-ODN or squalene-comprising emulsion.
3) An adjuvant composition comprising a peptide nucleic acid to which a cell-penetrating peptide is bound and CpG-ODN or squalene-comprising emulsion.
4) The composition of 3), comprising a pharmaceutically acceptable carrier.
5) A vaccine composition comprising the composition of 3) or 4) and an antigen.
6) Use of a peptide nucleic acid to which a cell-penetrating peptide is bound for producing an adjuvant activity enhancer for CpG-ODN or squalene-comprising emulsion.
7) A peptide nucleic acid to which a cell-penetrating peptide is bound, for enhancing an adjuvant activity of CpG-ODN or squalene-comprising emulsion.

### Advantageous Effect of Invention

The present invention can enhance the effectiveness of a vaccine, and largely contribute to the pharmaceutical industry as a creation of a preventive drug with high quality.

### Brief Description of Drawings

[Figure 1] Figure 1 shows IgG antibody titers against A/Kansas/14/2017 strain (A/H3N2) on Day 21.
[Figure 2] Figure 2 shows IgG antibody titers against B/Maryland/15/2016 strain (B/Victoria) on Day 21.
[Figure 3A] Figure 3A shows IgG1 subclass antibody titers against A/Kansas/14/2017 strain (A/H3N2) on Day 21.
[Figure 3B] Figure 3B shows IgG2a subclass antibody titers against A/Kansas/14/2017 strain (A/H3N2) on Day 21.
[Figure 3C] Figure 3C shows a subclass ratio specific to A/Kansas/14/2017 strain (A/H3N2) (IgG1/IgG2a).
[Figure 4A] Figure 4A shows IgG1 subclass antibody titers against B/Maryland/15/2016 strain (B/Victoria) on Day 21.
[Figure 4B] Figure 4B shows IgG2a subclass antibody titers against B/Maryland/15/2016 strain (B/Victoria) on Day 21.
[Figure 4C] Figure 4C shows a subclass ratio specific to B/Maryland/15/2016 strain (B/Victoria) (IgG1/IgG2a).

### Description of Embodiments

Preferred embodiments of the present invention will be described in detail below. However, the present invention is not limited to the following embodiments.

In the present invention, the phrase "peptide nucleic acid to which a cell-penetrating peptide is bound" means a molecule to which a peptide nucleic acid is bound at the N-terminal or C-terminal of a cell-penetrating peptide, and is also referred herein to as mitsumata-immunostimulatory peptide nucleic acid (MIP).

The phrase "cell-penetrating peptide (CPP)" refers to a peptide which passes through a cell membrane and translocates into the cell when coexisting with the cell. Examples thereof include, but not limited to, HIV TAT-derived peptides, Penetratin derived from Drosophila Antennapedia, polyarginine, Transportan derived from neuropeptide galanin, Pep-1 derived from an SV40 nuclear localization signal (NLS), and antimicrobial peptides LL37, shown in Table 1.

Preferable examples thereof include the TAT-derived peptides (peptides consisting of an amino acid sequence set forth in SEQ ID NO: 1).

**[Table 1]**

| Peptide | Origin | Sequence (N-terminal→C-terminal) |
|---|---|---|
| TAT(48-60) | HIV TAT protein | GRKKRRQRRRPPQ(SEQ ID NO: 1) |
| Penetratin | Drosophila Antennapedia | RQIKIWFQNRRMKWKK(SEQ ID NO: 2) |
| Polyarginine (R8) | Synthetic peptide | RRRRRRRR(SEQ ID NO: 3) |
| Polyarginine (R9) | Synthetic peptide | RRRRRRRRR(SEQ ID NO: 4) |
| Transportan | Neuropeptide Galanin | GWTLNSAGYLLGKINLKALAALAKKIL(SEQ ID NO: 5) |
| Pep-1 | SV40 NLS | KETWWETWWTEWSQPKKRKV(SEQ ID NO: 6) |
| LL-37 | Antimicrobial peptide | LLGDFERKSKEKIGKEFKRIVQRIKDFLRNLV PRTESC(SEQ ID NO: 7) |

The term "peptide nucleic acid (PNA)" is a nucleic acid analog which has a backbone in which the sugar-phosphate backbone of the nucleic acid is replaced by N-(2-aminoethyl)glycine, and to which the bases are bound by a methylenecarbonyl bond. Any of adenine, guanine, thymine, cytosine, and uracil may be used as a base in the peptide nucleic acid in the present invention. The chain length thereof is desirably 20-mer or less, which is common, and in view of adjuvant activity, preferably from 3 to 12-mer, and more preferably from 3 to 10-mer.

The following formula (1) shows the structure of a peptide nucleic acid ranging from 3 to 12-mer. 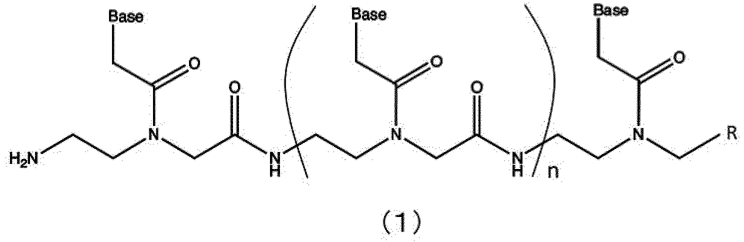 wherein, the bases may be the same or different, and represent adenine, guanine, thymine, cytosine, or uracil, R represents -COOH or -CONH₂, and n represents an integer from 1 to 10.

Examples of the suitable peptide nucleic acid used in the present invention include a 3-mer or 10-mer peptide nucleic acid having at least one guanine or cytosine as a base, a 3-mer peptide nucleic acid having three guanines (G3PNA) as a base, a 3-mer peptide nucleic acid having three cytosines (C3PNA) as a base, and a 10-mer peptide nucleic acid having ten cytosines (C10PNA) as a base.

The peptide nucleic acids to which a cell-penetrating peptide is bound of the present invention can be produced by covalently binding between the N-terminals of the cell-penetrating peptide and the peptide nucleic acid to the N- or C-terminal of the cell-penetrating peptide by a chemical approach, for example, via a crosslinker with N-hydroxysuccinimide ester at both ends.

Note that the peptide nucleic acid can be synthesized by using a solid-phase peptide synthesis method known in the art, for example, with use of an Fmoc-type PNA monomer unit.

Examples of the peptide nucleic acid to which a cell-penetrating peptide is bound suitable for use in the present invention include MIP01 (formula (2)) and MIP03 (formula (3)), in which an HIV TAT-derived peptide consisting of 13 amino acids (SEQ ID NO: 1) described below is covalently bound at its C-terminal to G3PNA and C10PNA, respectively.

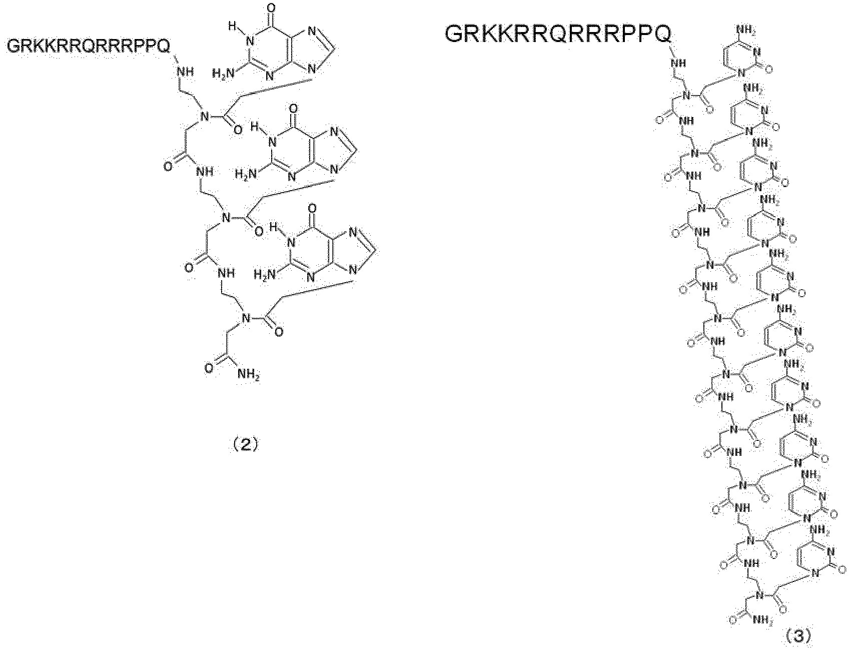

According to the present invention, the CpG-ODN or squalene-containing emulsion used in combination with the above-described peptide nucleic acid to which a cell-penetrating peptide is bound is a molecule or composition, both of which are known to be used as an adjuvant.

The CpG-ODN (CpG oligonucleotide) is a synthesized nucleic acid comprising a CpG motif (oligo-DNA composed of specific 6 bases (5'-purine-purine-CG-pyrimidine-pyrimidine-3')), and is known as a potent Th1 adjuvant which induces IFN Type I, inflammatory cytokines, proliferation of B-cells, maturation of dendritic cells, activation of NK cells, and the like via TLR9. The CpG-ODN is classified into 3 classes of A to C depending on the structural features, and the sequence structure of the CpG-ODN of the present invention is not limited, but the B-class CpG-ODN is preferred in view of the induction of the proliferation of B-cells and the maturation of dendritic cells.

The structure of the B-class CpG-ODN is characterized by being linear and having a backbone of phosphorothioate, and for example, ODN1668 (5'-tccatgacgttcctgatgct-3' (SEQ ID NO: 8)), ODN1826 (5'-tccatgacgttcctgacgtt-3' (SEQ ID NO: 9)), ODN2006 (5'-tcgtcgttttgtcgttttgtcgtt-3' (SEQ ID NO: 10)), and CpG1018 (5'-tgactgtgaacgttcgagatga-3' (SEQ ID NO: 11)) are commercially available.

The squalene-containing emulsion is an oil-in-water emulsion containing from 3 to 15 mg, preferably from 5 to 10 mg of squalene per dose and may contain a surfactant and an antioxidant such as tocopherol besides squalene. Examples of commercial products thereof include Addavax (trademark) (from InvivoGen; hereinafter referred to as "Addavax").

In the combination of the peptide nucleic acid to which a cell-penetrating peptide is bound and the CpG-ODN or squalene-containing emulsion of the present invention, the ratio is preferably in the range of from 0.06 to 60 parts by mass, and more preferably from 0.6 to 30 parts by mass of the CpG-ODN to be used, per 1 part by mass of the peptide nucleic acid to which a cell-penetrating peptide is bound. For the squalene-containing emulsion, the content is preferably in the range of from 30 to 150 parts by mass, and more preferably from 50 to 100 parts by mass.

As shown in Examples described later, in a case where a vaccine composition prepared by combining MIP03 of formula (3) with the CpG-ODN (ODN1826) or the squalene-containing emulsion (Addavax), and adding an influenza antigen (split antigen) to each thereof, is injected into mice, the blood antibody titer against the antigen synergistically increases. Further, in the evaluation of IgG subclasses, as for the induction balance between Th1 immunity and Th2 immunity, the respective properties of the CpG-ODN and the squalene-containing emulsion are maintained. That is, the combination of the peptide nucleic acid to which a cell-penetrating peptide is bound with the CpG-ODN or squalene-containing emulsion results in having a function of enhancing the immune strength of the adjuvant, without changing the quality of immunity which is induced by the CpG-ODN or squalene-containing emulsion.

Accordingly, the peptide nucleic acid to which a cell-penetrating peptide is bound is an adjuvant activity enhancer for the CpG-ODN or squalene-containing emulsion, and use of the peptide nucleic acid to which a cell-penetrating peptide is bound in combination with the CpG-ODN or squalene-containing emulsion can enhance the adjuvant activity of the CpG-ODN or squalene-containing emulsion. Moreover, the composition containing the peptide nucleic acid to which a cell-penetrating peptide is bound and the CpG-ODN or squalene-containing emulsion can be an adjuvant composition which exhibits an enhanced immune strength.

In the present invention, the term "adjuvant" means a substance for increasing an immune response to an antigen when the antigen is administered by injection or via mucosa.

In the present invention, the phrase "enhancement of the adjuvant activity of CpG-ODN or squalene-containing emulsion" means the enhancement of the immune strength of the CpG-ODN or squalene-containing emulsion (adjuvant substance) without changing the quality of immunity which is induced by the adjuvant substance.

The adjuvant composition of the present invention can be administered in combination with an antigen, and the administration thereof may be concurrent with, before or after the administration of the antigen.

The amount of the adjuvant composition of the present invention to be administered can be appropriately determined according to the subject to be administered, the dosing method, the dosage form, and the type of the antigen.

The adjuvant composition of the present invention can be combined with the antigen to form a vaccine composition, and a pharmaceutically acceptable carrier can also be added thereto appropriately to form an adequate formulation. In the vaccine composition of the present invention, the peptide nucleic acid to which a cell-penetrating peptide is bound (MIP) may be in a chemically bound state with the antigen or other components, or may be present in a free molecular state.

The antigen corresponds to a natural product purified from a pathogen, or a protein or peptide artificially made by a technique such as gene recombination, specific examples thereof include virions, which are entire virus particles, incomplete virus particles, viral structural proteins, viral non-structural proteins, whole pathogenic bacterial cells, proteins or glycoproteins derived from pathogenic bacteria, infection-protective antigens, and neutralizing epitopes, and both those with infectivity and those that have lost infectivity (inactivated antigen) are included. Examples of the inactivated antigen include, but not limited to, those inactivated by an operation, for example, physically (X-ray irradiation, UV irradiation, heat, ultrasonic waves), or chemically (formalin, beta-propiolactone, binary ethylenimine, mercury, alcohol, chlorin). The antigen derived from a pathogen is desirably an inactivated antigen derived from the above-described virus or pathogenic bacteria from the viewpoint of safety.

Examples of the virus include measles viruses, rubella viruses, mumps viruses, polioviruses, rotaviruses, noroviruses, adenoviruses, enteroviruses, herpes viruses, varicella viruses, severe acute respiratory syndrome (SARS) viruses, human immunodeficiency viruses (HIV), human T-cells leukemia viruses (HTLV-1), human papillomaviruses, Japanese encephalitis viruses, West Nile viruses, yellow fever viruses, dengue viruses, Zika viruses, hepatitis A viruses, hepatitis B viruses, hepatitis C viruses, hepatitis E viruses, RS viruses, and influenza viruses, and the virus is preferably an influenza virus.

Examples of the pathogenic bacteria include Corynebacterium diphtheriae, Clostridium tetani, Bordetella pertussis, Neisseria meningitidis, Haemophilus influenzae type b, Streptococcus pneumoniae, Vibrio cholerae, Mycobacterium tuberculosis, and periodontal bacteria.

Examples of dosage forms of the vaccine composition include liquid, suspension, and a freeze-dry formulation. Examples of the liquid include those dissolved in purified water or buffer. Examples of the suspension include those obtained by suspending in purified water, buffer, or the like with methylcellulose, hydroxymethylcellulose, polyvinylpyrrolidone, gelatin, casein, or the like.

Added to these formulations as necessary are an absorption promoter, a surfactant, a preservative, a stabilizing agent, an anti-moisture agent, a solubilizer, or the like, which are commonly used.

The vaccine composition of the present invention can also contain an adjuvant substance other than the peptide nucleic acid to which a cell-penetrating peptide is bound and the CpG-ODN and squalene-containing emulsion as long as the substance does not diminish the immunogenicity and safety of the vaccine.

The amount of the antigen contained in the vaccine composition of the present invention is not particularly limited as long as it is enough to induce a specific antibody response, and may be appropriately set in consideration of the ratio thereof to the adjuvant composition used in combination. For example, when the split antigen of an influenza virus is used as the antigen, the content thereof may be within a range of from 1 to 60 µg HA (in terms of HA), which is a single dose, and the range of from 9 to 15 µg HA (in terms of HA) is more preferable. The concentration is a value obtained by measuring the concentration of HA protein by a testing method defined in accordance with WHO or national standards, such as the single radial immunodiffusion.

The administration route of the vaccine composition of the present invention is not particularly limited, any of injection administration (subcutaneous, intramuscular, intradermic, intravenous administration), oral administration, and parenteral administration (e.g., nasal, ophthalmic, transvaginal, sublingual, transdermal administration) can be performed, and for example, the composition is administered into a nasal cavity by dropping, nebulizing, or spraying.

Examples of the subject to which the adjuvant composition or vaccine composition of the present invention is administered include humans and mammals except humans, and the humans are preferable. Examples of the mammals except humans include mice, rats, hamsters, guinea pigs, rabbits, pigs, cows, goats, sheep, dogs, cats, rhesus macaques, cynomolgus monkey, orangutans, and chimpanzees.

### Examples

The present invention will now be more specifically described with reference to Examples; however, the present invention is not limited to these.

### <Peptide Nucleic Acid to Which Cell-Penetrating Peptide is Bound (MIP)>

Synthesis of MIP03 of formula (3) as MIP was entrusted to PEPTIDE INSTITUTE, INC.

### Example 1: Evaluation of Adjuvant Activity of DIF in Influenza Vaccine

Each undiluted solution of A/H1N1 subtype (A/Kansas/14/2017 strain) and B/Victoria lineage (B/Maryland/15/2016 strain) of an influenza HA vaccine "Seiken" was used as a split antigen (SV), and 0.6 µg of hemagglutinin of each strain, 100 µg of MIP03, and 6 µg of CpG-ODN (ODN1826 (InvivoGen); SEQ ID NO: 9) were added thereto per 0.1 mL to obtain a DIF-01 formulation. Further, the split antigen (SV) was used and 0.6 µg of hemagglutinin of each strain and 100 µg of MIP03 were added thereto per 0.05 mL, and an equal amount of aluminum hydroxide (Alm (Thermo Scientific)) or squalene-containing emulsion (Addavax (InvivoGen)) was mixed into the solution prepared just above to obtain a DIF-02 formulation and a DIF-03 formulation, respectively.

As controls, a non-adjuvanted formulation prepared so that hemagglutinin of each strain was 0.6 µg per 0.1 mL, an MIP-03 formulation prepared so that hemagglutinin of each strain was 0.6 µg and MIP03 was 100 µg per 0.1 mL, an ODN1826 formulation prepared so that hemagglutinin of each strain was 0.6 µg and ODN1826 was 6 µg per 0.1 mL, and an Alm formulation and an Addavax formulation each prepared by using the split antigen (SV) so that hemagglutinin of each strain was 0.6 µg per 0.05 mL and mixing an equal amount of Alm (Thermo Scientific) or Addavax (InvivoGen) thereto were each prepared.

The formulations prepared as described above (Table 2) were administered subcutaneously in the posterodorsal part of BALB/c mice (female, 5 weeks of age) at an amount of 0.1 mL (8 mice per group), and whole blood was collected 21 days after the administration. The blood obtained from the whole blood collection was centrifuged to prepare serum, and then IgG (Total IgG) antibody titers in which the serum is specifically bound to the A/Kansas/14/2017 strain and the B/Maryland/15/2016 strain, IgG1 subclass antibody titers and IgG2a subclass antibody titers were each measured.

**[Table 2]**

| Formulation No. | Antigen | | Adjuvant | |
|---|---|---|---|---|
| | Subtype·lineage | Dose (µg HA/strain/shot) | Name | Contained substance |
| 1 | A/H3N2 B/Victoria | 0.6 | DIF-01 | MIP-03 ODN1826 |
| 2 | A/H3N2 B/Victoria | 0.6 | DIF-02 | MIP-03 Alm (aluminum hydroxide) |
| 3 | A/H3N2 B/Victoria | 0.6 | DIF-03 | MIP-03 Addavax |
| 4 | A/H3N2 B/Victoria | 0.6 | MIP-03 | MIP-03 |
| 5 | A/H3N2 B/Victoria | 0.6 | ODN1826 | ODN1826 |
| 6 | A/H3N2 B/Victoria | 0.6 | Aim | Alm (aluminum hydroxide) |
| 7 | A/H3N2 B/Victoria | 0.6 | Addavax | Addavax |
| 8 | A/H3N2 B/Victoria | 0.6 | Non-adjuvanted | - |

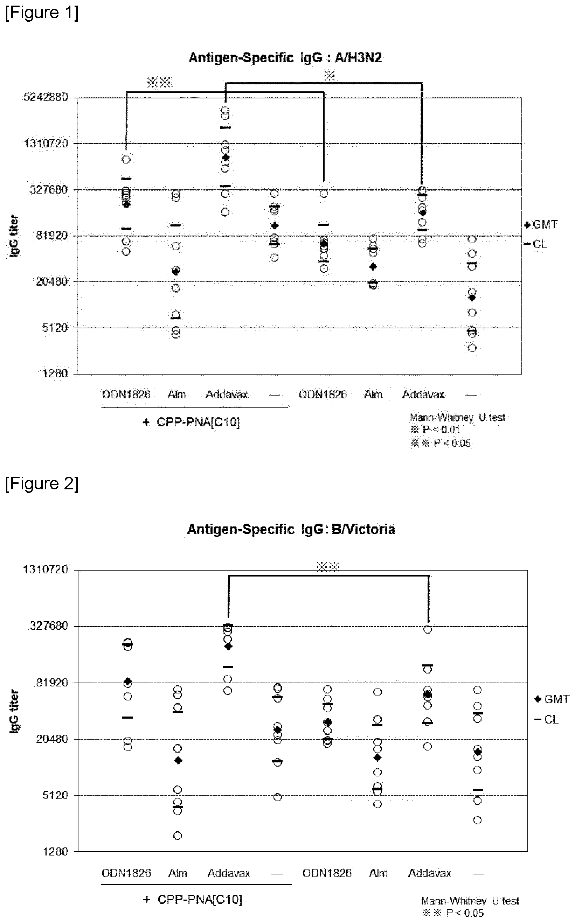

The IgG antibody titers of each treatment group are as shown in Figures 1 and 2. The antibody titers against the A/Kansas/14/2017 strain showed antibody induction significantly higher in the DIF-01 formulation treatment group over in the ODN1826 formulation treatment group and higher in the DIF-03 formulation treatment group over the Addavax formulation treatment group, respectively. The antibody titers against the B/Maryland/15/2016 strain showed antibody induction significantly higher in the DIF-03 formulation treatment group over the Addavax formulation treatment group, and the antibody titers were higher in the DIF-01 formulation treatment group over the ODN1826 formulation treatment group although there was no significant difference. Tables 3 and 4 summarize the geometric mean antibody titers (GMT) of DIF-01 and DIF-03 which showed statistically significant differences, and single treatment groups of the adjuvants contained in the DIF-01 and DIF-03, and the ratio of GMT to the non-adjuvanted group. Table 3 summarizes the GMT and GMT ratio for the A/Kansas/14/2017 strain, and the DIF-01 has a GMT ratio higher than those of the ODN1826 and MIP-03, which is considered to exhibit a synergistic effect, and this was also the case in comparison of the DIF-03 to Addavax and MIP-03. As for the B/Maryland/15/2016 strain, similar to the A/Kansas/14/2017 strain, the GMTs of the DIF-01 and DIF-03 are considered to be a result of synergistic adjuvant effects as shown in Table 4. Accordingly, the DIF-01 and DIF-03 exhibited a synergistic immunostimulatory effect by mixing the adjuvants. Meanwhile, it was found that antibody induction of the DIF-02 formulation treatment group and the Alm formulation treatment group was in similar levels, and the MIP-03 cannot be expected to enhance the adjuvant activity when mixed with the Alm.

**[Table 3]**

| Adjuvant | GMT | GMT ratio* |
|---|---|---|
| DIF-01 | 212924 | 16.6 |
| ODN1826 | 65838 | 5.1 |
| DIF-03 | 870079 | 67.7 |
| Addavax | 163364 | 12.7 |
| MIP-03 | 112071 | 8.7 |
| Non-Adjuvanted | 12860 | 1.0 |

| | | |
|---|---|---|
| *GMT ratio to the non-adjuvanted group | | |

**[Table 4]**

| Adjuvant | GMT | GMT ratio* |
|---|---|---|
| DIF-01 | 84733 | 5.7 |
| ODN1826 | 31152 | 2.1 |
| DIF-03 | 201740 | 13.5 |
| Addavax | 61167 | 4.1 |
| MIP-03 | 25802 | 1.7 |
| Non-Adjuvanted | 14923 | 1.0 |

| | | |
|---|---|---|
| *GMT ratio to the non-adjuvanted group | | |

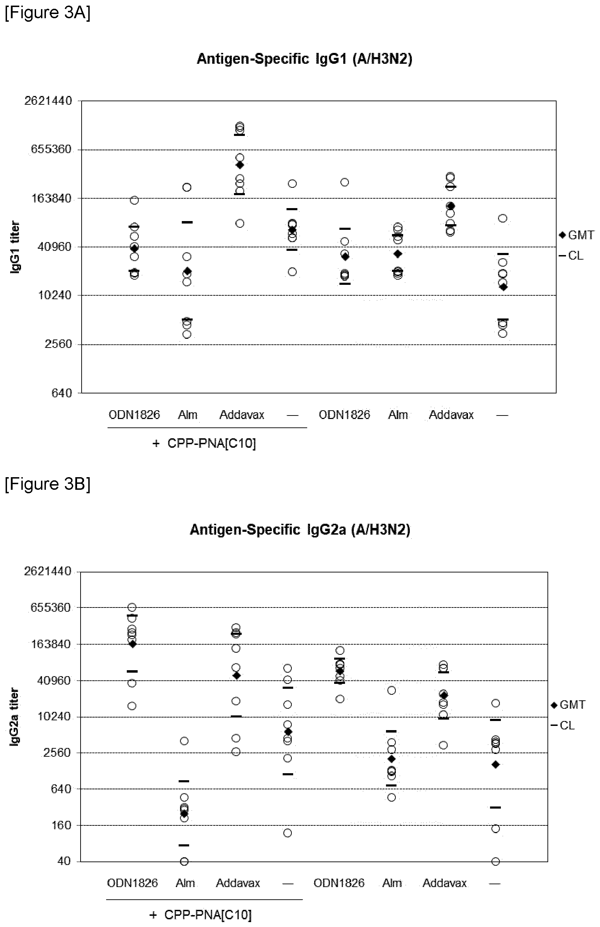

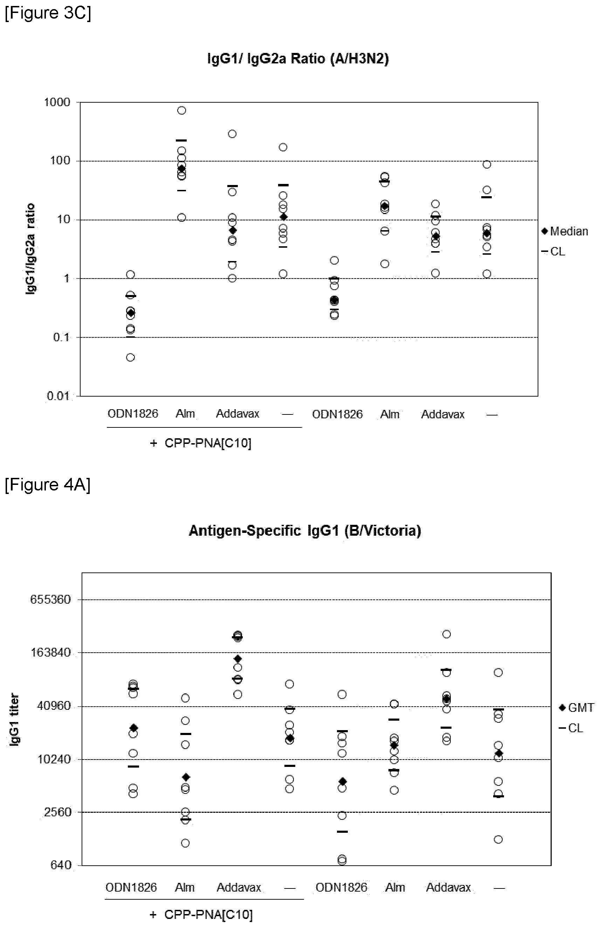

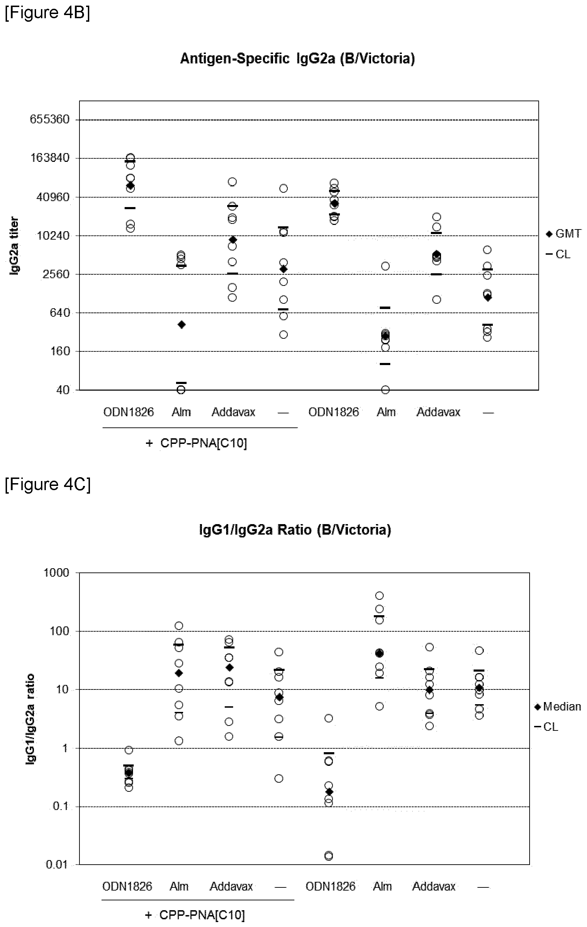

Next, the antibody titers and subclass ratios of the IgG1 and IgG2a subclasses were evaluated. The results of the A/Kansas/14/2017 strain are shown in Figures 3A to 3C, and the results of the B/Maryland/15/2016 strain are shown in Figures 4A to 4C. The antibody titers of each subclass of the DIF-01 formulation and the DIF-03 treatment group are shown as antibody titers equal to or higher than those of the ODN1826 formulation treatment group and the Addavax formulation treatment group, but the subclass ratio of IgG1/IgG2a in the DIF-01 formulation treatment group is IgG2a dominant similar to the ODN1826 formulation treatment group, and that in the DIF-03 formulation treatment group is IgG1 dominant similar to the Addavax formulation treatment group. Accordingly, the MIP-03 is considered to have an effect of enhancing the immune strength without changing the quality of immunity that is induced by known adjuvants to be mixed.

## Claims

1. An adjuvant activity enhancer for CpG-ODN or squalene-comprising emulsion, having an active ingredient of a peptide nucleic acid to which a cell-penetrating peptide is bound.

2. A method for enhancing an adjuvant activity of CpG-ODN or squalene-comprising emulsion, wherein a peptide nucleic acid to which a cell-penetrating peptide is bound is used in combination with the CpG-ODN or squalene-comprising emulsion.

3. An adjuvant composition comprising a peptide nucleic acid to which a cell-penetrating peptide is bound and CpG-ODN or squalene-comprising emulsion.

4. The composition of Claim 3, comprising a pharmaceutically acceptable carrier.

5. A vaccine composition comprising the composition of Claim 3 or 4 and an antigen.

6. Use of a peptide nucleic acid to which a cell-penetrating peptide is bound for producing an adjuvant activity enhancer for CpG-ODN or squalene-comprising emulsion.

7. A peptide nucleic acid to which a cell-penetrating peptide is bound, for enhancing an adjuvant activity of CpG-ODN or squalene-comprising emulsion.
